# EUROPEAN PATENT APPLICATION

(11) **EP 3 437 809 A1**
(43) Date of publication of application: **06.02.2019**
(21) Application number: 17775156.7
(22) Date of filing: 28.03.2017
(51) Int. Cl.: B25J 11/00, A61F 2/54

(54) **MUSCLE-POWER SUPPORT DEVICE**

(30) Priority: 30.03.2016 JP 2016068867
(71) Applicant: National University Corporation Kagawa University, Takamatsu-shi, Kagawa 760-8521 (JP); Nabtesco Corporation, Tokyo 102-0093 (JP)
(72) Inventor: SASAKI Daisuke, Takamatsu-shi Kagawa 761-0396 (JP); SAKAMOTO Takashi, Tokyo 102-0093 (JP); KIKUTANI Isao, Tokyo 102-0093 (JP)
(74) Representative: Grünecker Patent- und Rechtsanwälte PartG mbB
(86) International application number: PCT/JP2017/012771
(87) International publication number: WO 2017/170619

(57) **Abstract**

A muscular strength assisting apparatus (10) includes a first attachment tool (20) mounted to one of two parts of a human body, which are connected together via a joint, a second attachment tool (30) mounted to the other part of the human body, and a rotary member (50) rotatably supported to the first attachment tool and connected to the second attachment tool. A force for stopping rotation or rotating with respect to the first attachment tool is exerted on the rotary member. The second attachment tool includes an attachment portion (31) attached to the human body and a coupling portion (33) coupling the rotary member to the attachment portion. In a plane orthogonal to a rotation axis (d1) of the rotary member and including a connection position (dj) between the attachment portion and the coupling portion, a distance between a connection position (pj) of the coupling portion to the rotary member and the connection position (dj) of the coupling portion to the attachment portion varies.

## Description

### TECHNICAL FIELD

The present invention relates generally to a muscular strength assisting apparatus that is worn by a wearer so as to assist muscular strength of the wearer and particularly to a muscular strength assisting apparatus capable of reducing a restraint feeling of the wearer.

### BACKGROUND ART

As disclosed in, for example, Japanese Patent Application Publication No. 2009-268839A, attention has been focused on a muscular strength assisting apparatus with the aim of reducing a muscular strain on workers engaged in agriculture, construction industry, nursing care, and so on. A muscular strength assisting apparatus includes a pair of attachment tools mounted respectively to two parts of a human body, which are connected together via a joint, and assists muscular strength of a wearer of the muscular strength assisting apparatus by retaining the pair of attachment tools at a predetermined relative position or by actively causing the pair of attachment tools to operate relative to each other.

In order to efficiently exert a muscular strength assisting force from the muscular strength assisting apparatus to the human body, it is required to align an axis of relative rotation between the pair of attachment tools with a rotation axis of the joint. It is, however, difficult to accurately align the rotation axis of the muscular strength assisting apparatus with the rotation axis of the joint. Furthermore, it is also assumable that the rotation axis of the muscular strength assisting apparatus, which must have been aligned beforehand with the rotation axis of the joint, becomes misaligned from the rotation axis of the joint while the wearer of the muscular strength assisting apparatus is at work. Then, once such misalignment happens between the rotation axis of the muscular strength assisting apparatus and the rotation axis of the joint, it becomes difficult to move the joint over the entire range of joint actions, resulting in giving a restraint feeling to the wearer.

### DISCLOSURE OF INVENTION

The present invention has been made in view of the above-described circumstances, and it is an object of the present invention to provide a muscular strength assisting apparatus capable of reducing a restraint feeling that a wearer of the muscular strength assisting apparatus might experience.

A muscular strength assisting apparatus according to the present invention is provided with a first attachment tool mounted to one of two parts of a human body, which are connected together via a joint, a second attachment tool mounted to the other part of the human body, and a rotary member rotatably supported to the first attachment tool and connected to the second attachment tool, the rotary member receiving a force for stopping or rotating with respect to the first attachment tool. The second attachment tool includes an attachment portion attached to the human body and a coupling portion coupling the rotary member to the attachment portion so that the attachment portion rotates with respect to the first attachment tool as the rotary member rotates with respect to the first attachment tool. In a plane orthogonal to a rotation axis of the rotary member and including a connection position between the attachment portion and the coupling portion, a distance between the rotation axis of the rotary member and the connection position of the coupling portion to the attachment portion varies.

In the muscular strength assisting apparatus according to the present invention, the rotary member, the coupling portion, and the attachment portion may constitute a mechanism having three degrees of freedom.

In the muscular strength assisting apparatus according to the present invention, the coupling portion may include a first member operably connected to the rotary member and a second member operably connected to the first member and operably connected to the attachment portion.

In the muscular strength assisting apparatus according to the present invention, a connection may be made in any one of following combinations: the rotary member and the first member of the coupling portion; the first member of the coupling portion and the second member of the coupling portion; and the second member of the coupling portion and the attachment portion, so as to enable rotation about an axis non-parallel to the rotation axis of the rotary member.

In the muscular strength assisting apparatus according to the present invention, relative rotation between the first attachment tool and the second attachment tool about the rotation axis of the rotary member may enable a flexion motion or an extension motion using the joint, and relative rotation of the one of the combinations about the axis non-parallel to the rotation axis of the rotary member may enable an adduction motion or an abduction motion using the joint.

In the muscular strength assisting apparatus according to the present invention, the first attachment tool may include a first portion and a second portion connected so as to be rotatable with respect to the first portion, and a rotation axis between the first portion and the second portion may be non-parallel to the rotation axis of the rotary member and also non-parallel to the rotation axis of the one of the combinations.

In the muscular strength assisting apparatus according to the present invention, the rotary member and the first member of the coupling portion may be rotatably connected to each other, the first member of the coupling portion and the second member of the coupling portion may be rotatably connected to each other, and the second member of the coupling portion and the attachment portion may be rotatably connected to each other.

In the muscular strength assisting apparatus according to the present invention, in a plane including the rotation axis of the rotary member and the connection position between the attachment portion and the coupling portion, a connection position between the first member and the second member of the coupling portion may be located on one side with respect to a straight line passing through a connection position of the coupling portion to the rotary member and the connection position of the coupling portion to the attachment portion.

In the muscular strength assisting apparatus according to the present invention, the coupling portion may include a fluid pressure cylinder.

In the muscular strength assisting apparatus according to the present invention, the connection position of the coupling portion to the rotary member may be located on the rotation axis of the rotary member.

A muscular strength assisting apparatus of another aspect according to the present invention is provided with a rotary member which is rotatable with respect to a first attachment tool and is connected to a second attachment tool, the first attachment tool being mounted to one of two parts of a human body which are connected together via a joint, the second attachment tool being mounted to the other part of the human body, a retention portion maintained in a state where relative rotation with respect to the rotary member is restricted and in a state where the relative rotation with respect to the rotary member is permitted, the retention portion being capable of restricting the relative rotation with respect to the rotary member, and an operation portion for acting, in the state where the relative rotation between the retention portion and the rotary member is restricted, on the retention portion so that the rotary member rotates together with the retention portion with respect to the first attachment tool.

According to the present invention, it is possible to reduce a restraint feeling that a wearer wearing the muscular strength assisting apparatus might receive from the muscular strength assisting apparatus.

According to the present invention, it is possible to fulfill an excellent muscular strength assisting function while reducing a restraint feeling that might be given to a wearer of the muscular strength assisting apparatus.

### BRIEF DESCRIPTION OF DRAWINGS

Fig. 1 is a perspective view showing a muscular strength assisting apparatus together with a wearer wearing the same for explaining one embodiment of the present invention.
Fig. 2 is a perspective view showing the muscular strength assisting apparatus shown in Fig. 1.
Fig. 3 is a view showing, from a forward side, part of the muscular strength assisting apparatus shown in Fig. 1.
Fig. 4 is a view showing, from a lateral outer side, the muscular strength assisting apparatus shown in Fig. 1.
Fig. 5 is a view showing, from the lateral outer side, the muscular strength assisting apparatus in a state different from a state shown in Fig. 4.
Fig. 6 is a view showing, from the lateral outer side, the muscular strength assisting apparatus in a state different from the states shown in Fig. 4 and Fig. 5, respectively.
Fig. 7 is a view showing, from the lateral outer side, the muscular strength assisting apparatus in a state different from the states shown in Fig. 4 to Fig. 6, respectively.
Fig. 8 is a view showing, from the lateral outer side, a main part of a driver of the muscular strength assisting apparatus in the state shown in Fig. 5.
Fig. 9 is a view showing, from the lateral outer side, the main part of the driver of the muscular strength assisting apparatus in the state shown in Fig. 6.
Fig. 10 is a view showing, from the lateral outer side, the main part of the driver of the muscular strength assisting apparatus in the state shown in Fig. 7.
Fig. 11 is a view showing, from the front, a second attachment tool of the muscular strength assisting apparatus in the state shown in Fig. 4.
Fig. 12 is a view showing, from above, the second attachment tool of the muscular strength assisting apparatus in the state shown in Fig. 5.
Fig. 13 is a view showing, from the front, the second attachment tool of the muscular strength assisting apparatus in a state where an arm is abducted from the state shown in Fig. 4.
Fig. 14 is a mechanism diagram schematically showing one modification example of the second attachment tool of the muscular strength assisting apparatus.
Fig. 15 is a mechanism diagram schematically showing another modification example of the second attachment tool of the muscular strength assisting apparatus.
Fig. 16 is a mechanism diagram schematically showing still another modification example of the second attachment tool of the muscular strength assisting apparatus.
Fig. 17 is a mechanism diagram schematically showing yet still another modification example of the second attachment tool of the muscular strength assisting apparatus.

### MODE FOR CARRYING OUT INVENTION

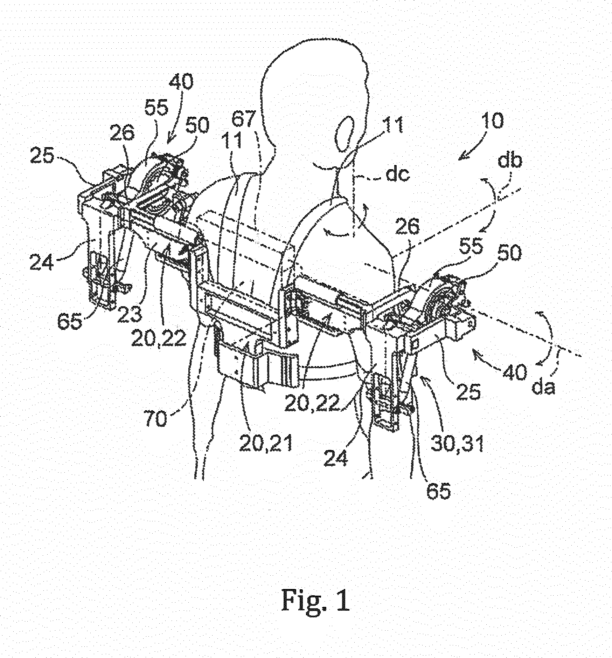

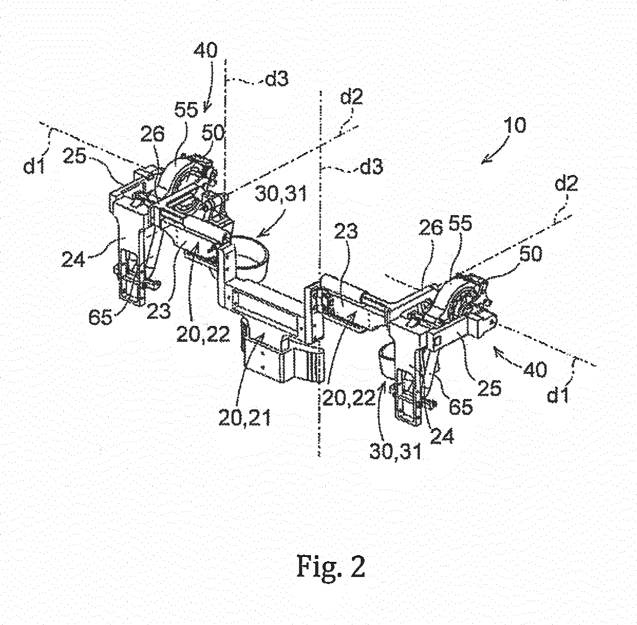

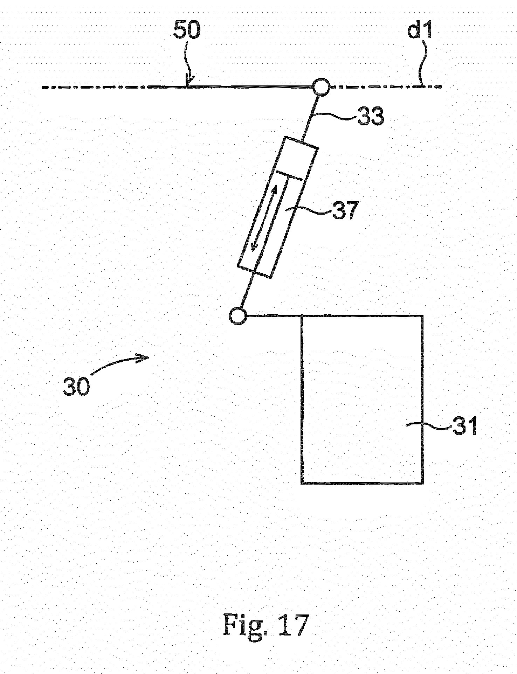

Hereinafter, one embodiment of the present invention will be described with reference to the appended drawings. Fig. 1 to Fig. 17 are views for explaining one embodiment of the present invention and modification examples thereof. Among these drawings, Fig. 1 and Fig. 2 are perspective views showing a muscular strength assisting apparatus 10 in its entirety, and Fig. 1 in particular shows a state where the muscular strength assisting apparatus 10 is worn on a human body. The muscular strength assisting apparatus 10 includes a first attachment tool 20 and a second attachment tool 30 rotatable relative to each other about a first rotation axis d1 and a driver 40 that controls a relative position between the first attachment tool 20 and the second attachment tool 30, in other words, a rotation position of the second attachment tool 30 with respect to the first attachment tool 20. As shown in Fig. 1, the first attachment tool 20 and the second attachment tool 30 are mounted respectively to two parts of the human body, which are connected together via a joint. The driver 40 controls the relative position between the first attachment tool 20 and the second attachment tool 30 and assists muscular strength of the two parts connected via the joint. In particular, the muscular strength assisting apparatus 10 described herein is designed so as to fulfill an excellent muscular strength assisting function while reducing a restraint feeling that might be given to a wearer wearing the same.

In an example shown, the first attachment tool 20 is mounted to a torso via an attachment belt 11, and the second attachment tool 30 is mounted to each upper arm via an after-mentioned attachment portion 31. Further, the muscular strength assisting apparatus 10 assists actions of shoulders. Here, a single joint enables relative rotation motions about axes different from each other. As shown in Fig. 1, a shoulder joint enables a flexion/extension motion in which the upper arm is rotated relative to the torso about a flexion/extension axis da, an adduction/abduction motion in which the upper arm is rotated relative to the torso about an adduction/abduction axis db, and an internal/external rotation motion in which the upper arm is rotated relative to the torso about an internal/external rotation axis dc. Further, as will be mentioned later, the muscular strength assisting apparatus 10 shown assists in retaining the upper arm at a predetermined position so as to prevent the upper arm from coming down during a flexion motion and further assists such a flexion motion in which the upper arm is lifted forward.

Furthermore, in the example shown, the driver 40 has a rotary member 50 as an output portion of a drive force. The rotary member 50 is rotatable relative to the first attachment tool 20 about the first rotation axis d1. The rotary member 50 is connected to the second attachment tool 30. The first rotation axis d1 acting as a rotation axis of the rotary member 50 is provided so as to correspond to the flexion/extension axis da of the shoulders. The second attachment tool 30 rotates relative to the first attachment tool 20 about the first rotation axis d1, thus enabling a flexion motion and an extension motion of the upper arm about the shoulder joint.

The muscular strength assisting apparatus according to the present invention is not limited to the example shown and may assist any one or more of a flexion motion, an extension motion, an adduction motion, an abduction motion, an internal rotation motion, and an external rotation motion. The muscular strength assisting apparatus according to the present invention is not limited to the example shown and may assist motions of a human body at other parts thereof such as an elbow, a neck, a lower back, a crotch, or a wrist.

The constituent elements will now be described in order. First, a description is given of the first attachment tool 20. The first attachment tool 20 has a first portion 21 and a second portion 22. As shown in Fig. 1, the first portion 21 is provided with the attachment belt 11. The first portion 21 is attached to the torso of the wearer via the attachment belt 11. The second portion 22 is connected so as to be rotatable relative to the first portion 21 about a third rotation axis d3. The third rotation axis d3 is provided so as to correspond to the internal/external rotation axis dc of the shoulders. The second portion 22 rotates relative to the first portion 21 about the third rotation axis d3, thus enabling an internal rotation motion and an external rotation motion of the upper arm about the shoulder joint.

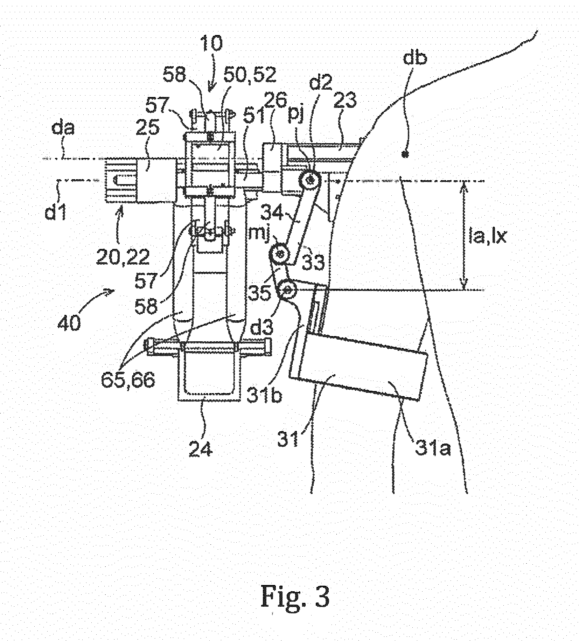

As shown in Fig. 2, the second portion 22 has a lateral frame 23, a vertical frame 24, an outer support arm 25, and an inner support arm 26. The lateral frame 23 is rotatably connected to the first portion 21. The lateral frame 23 extends outward in a transverse direction from the first portion 21. The vertical frame 24 extends downward from an outer end portion of the lateral frame 23. The outer support arm 25 and the inner support arm 26 extend forward from an upper end portion of the vertical frame 24. The outer support arm 25 and the inner support arm 26 extend substantially parallel to each other so as to be spaced from each other in the transverse direction. The outer support arm 25 is positioned on an outer side in the transverse direction beyond the inner support arm 26. In Fig. 3 to Fig. 7, for easier understanding, depiction of the outer support arm 25 is partially omitted.

The muscular strength assisting apparatus 10 shown, which assists actions of the shoulders, is configured to act on both the shoulders. Specifically, the second portion 22 is provided on each lateral side of the first portion 21. The rotary member 50 and the like of the driver 40 are supported with respect to each of the second portions 22. Furthermore, the second attachment tool 30 is connected to each of the second portions 22. A pair of constituent elements thus provided respectively on both lateral sides with respect to the first portion 21 are symmetrical to each other and yet can be configured in the same manner in other respects. The following description and Fig. 3 to Fig. 16, therefore, are based on a configuration in which the muscular strength assisting apparatus 10 acts on the right shoulder.

Furthermore, the terms used herein, such as "front," "rear," "upper," "lower," and "a transverse direction," are assumed to refer to corresponding directions "front," "rear," "upper," "lower," and "a transverse direction" with reference to the wearer wearing the muscular strength assisting apparatus 10.

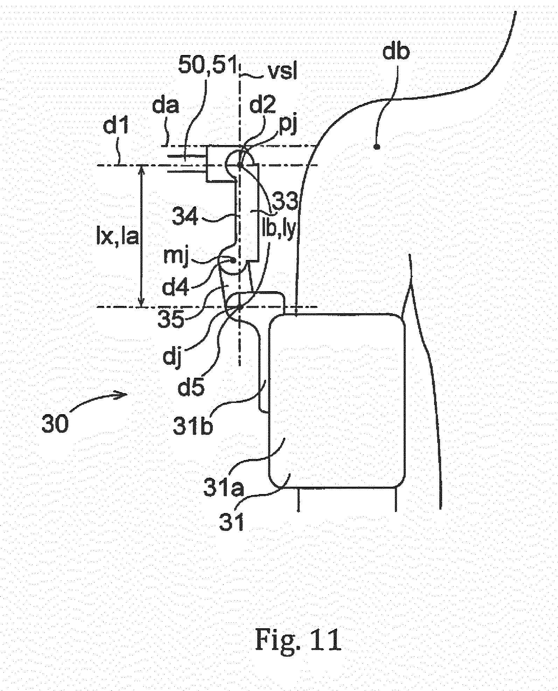

Next, a description is given of the second attachment tool 30. Fig. 3 and Fig. 11 show the second attachment tool 30 from a forward side (the front). The second attachment tool 30 is connected to the rotary member 50 of the driver 40, which will be detailed later. As the rotary member 50 rotates, the second attachment tool 30 rotates about the first rotation axis d1 that is the rotation axis of the rotary member 50. The second attachment tool 30 has the attachment portion 31 attached to the human body and a coupling portion 33 coupling the rotary member 50 to the second attachment tool 30. The coupling portion 33 couples the rotary member 50 to the attachment portion 31 so that, as the rotary member 50 rotates with respect to the first attachment tool 20, the attachment portion 31 rotates with respect to the first attachment tool 20.

In the example shown, the attachment portion 31 is attached to the upper arm of the human body. In order to efficiently transfer a force supplied from the driver 40 to the upper arm, preferably, the attachment portion 31 is configured so that relative movement with respect to the upper arm in a direction orthogonal to a direction of movement caused by rotation around the first rotation axis d1 is restricted. Meanwhile, for the purpose of reducing a restraint feeling that the wearer might experience, with respect to the upper arm, the attachment portion 31 may be configured so that relative movement with respect to the upper arm along a longitudinal direction of the upper arm and relative rotation with respect to the upper arm around a center axis of the upper arm are permitted to some extent. In the example shown, the attachment portion 31 has a cylindrical portion 31a for inserting the upper arm thereinto and a bracket 31b connected to the coupling portion 33. The cylindrical portion 31a and the bracket 31b are secured to each other.

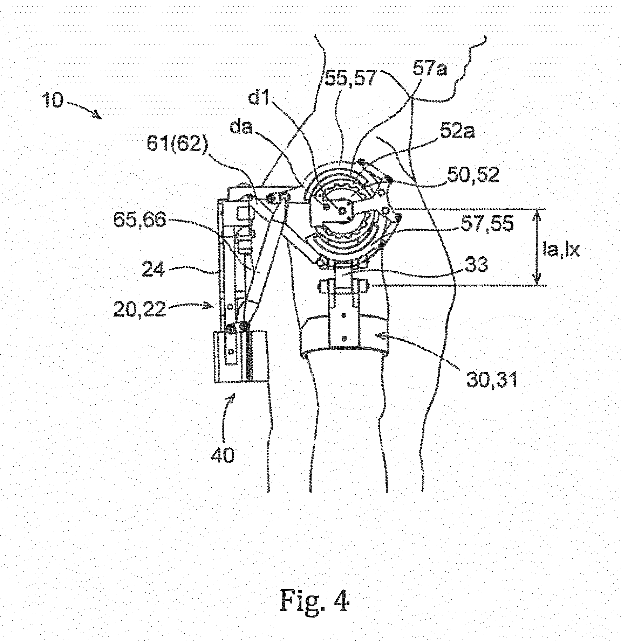

For the purpose of reducing a restraint feeling of the wearer, the coupling portion 33 is configured so that, in a plane orthogonal to the first rotation axis d1 that is the rotation axis of the rotary member 50 and including a connection position (hereinafter, referred to also as a "distal end side connection position") dj between the attachment portion 31 and the coupling portion 33, a distance la between a connection position (hereinafter, referred to also as a "proximal end side connection position") pj between the rotary member 50 and the coupling portion 33 and the distal end side connection position dj varies. Thus, in the plane orthogonal to the first rotation axis d1 and including the distal end side connection position dj, a distance Ix from the first rotation axis d1 to the distal end side connection position dj varies. Here, the plane orthogonal to the first rotation axis d1 and including the distal end side connection position dj is a plane parallel to a plane shown in Fig. 4 to Fig. 7. Further, the distance la from the proximal end side connection position pj to the distal end side connection position dj in the plane parallel to the plane shown in Fig. 4 to Fig. 7 can be defined also as a separation spacing between the first rotation axis d1 and the distal end side connection position dj along a direction orthogonal to the first rotation axis d1 in a plane including the first rotation axis d1 and the distal end side connection position dj.

Furthermore, for the purpose of reducing a restraint feeling of the wearer, preferably, a portion extending from the rotary member 50 to the attachment portion 31 via the coupling portion 33 constitute a mechanism having three degrees of freedom. In the example shown in Fig. 3 and Fig. 11, the coupling portion 33 has a first member 34 operably connected to the rotary member 50 and a second member 35 operably connected to the first member 34 and operably connected to the attachment portion 31. According to the coupling portion 33 thus described, the configuration extending from the rotary member 50 to the attachment portion 31 enables actions with three degrees of freedom. In other words, the attachment portion 31 can perform relative operations with three degrees of freedom with respect to the rotary member 50.

Moreover, preferably, any one of a connection between the rotary member 50 and the first member 34, a connection between the first member 34 and the second member 35, and a connection between the second member 35 and the attachment portion 31 is a connection allowing rotation about an axis non-parallel to the first rotation axis d1 that is the rotation axis of the rotary member 50. The connection allowing rotation about the axis non-parallel to the first rotation axis d1 would not interfere with the attachment portion 31 rotating about the first rotation axis d1 as the rotary member 50 rotates. On the other hand, in the example shown, this connection can enable the attachment portion 31 to move together with the upper arm, following an action using the shoulder joint about the axis non-parallel to the first rotation axis d1.

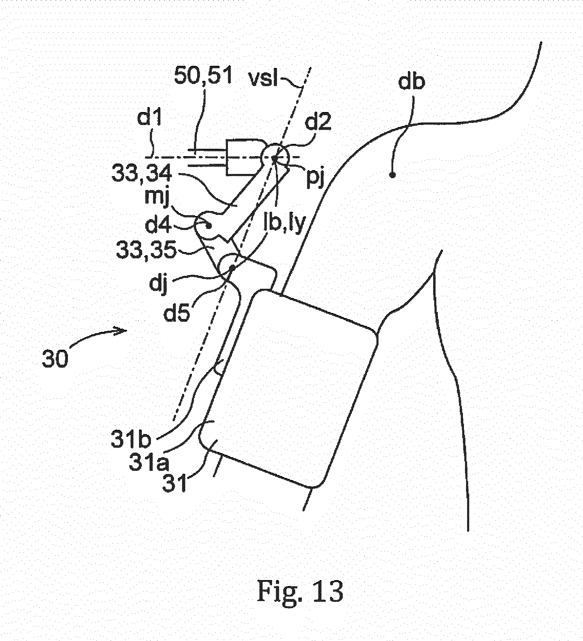

In the example shown, the rotary member 50 and the first member 34 are rotatably connected to each other. An axis of relative rotation between the rotary member 50 and the first member 34 is a second rotation axis d2 non-parallel to the first rotation axis d1. Particularly in the example shown, the second rotation axis d2 is orthogonal to the first rotation axis d1. Furthermore, the second rotation axis d2 is orthogonal also to the third rotation axis d3. Moreover, in the example shown, the proximal end side connection position pj that is the connection position of the coupling portion 33 to the rotary member 50 is located on the first rotation axis d1 that is the rotation axis of the rotary member 50. Therefore, in the plane orthogonal to the first rotation axis d1 and including the distal end side connection position dj, the distance la between the proximal end side connection position pj and the distal end side connection position dj agrees with the distance Ix between the first rotation axis d1 and the distal end side connection position dj. As mentioned above, relative rotation between the constituent elements about the first rotation axis d1 enables a flexion motion and an extension motion using the joint. Further, relative rotation between the rotary member 50 and the second attachment tool 30 about the second rotation axis d2 enables an adduction motion and an abduction motion using the joint as shown in Fig. 13.

Furthermore, in the example shown, the first member 34 and the second member 35 of the coupling portion 33 are connected to each other so as to be rotatable about a fourth rotation axis d4. The second member 35 of the coupling portion 33 and the attachment portion 31 are connected to each other so as to be rotatable about a fifth rotation axis d5. The fourth rotation axis d4 and the fifth rotation axis d5 are parallel to the second rotation axis d2. By this configuration, in a plane orthogonal to the second rotation axis d2 and including the distal end side connection position dj (Fig. 11 and Fig. 13), the second rotation axis d2 being provided so as to correspond to an adduction/abduction motion, a distance lb between the proximal end side connection position pj and the distal end side connection position dj is set to vary.

Furthermore, as shown in Fig. 11 to Fig. 13, in the plane including the first rotation axis d1 and the distal end side connection position dj, a connection position (hereinafter, referred to also as an "intermediate connection position") mj between the first member 34 and the second member 35 of the coupling portion 33 is always located on one side with respect to a virtual straight line vsl passing through the proximal end side connection position pj and the distal end side connection position dj. Particularly in the example shown, structurally, the intermediate connection position mj is set so as to be always on one side with respect to the virtual straight line vsl passing through the proximal end side connection position pj and the distal end side connection position dj. This means that, even in a case where the coupling portion 33 is extended so that both ends thereof are maximally separated from each other, the intermediate connection position mj is not located on the virtual straight line vsl. Further, in the example shown, the intermediate connection position mj is located on the same side as the rotary member 50 with respect to the virtual straight line vsl. As shown in Fig. 11 to Fig. 13, the intermediate connection position mj, together with the rotary member 50, is located on an opposite side to the human body with respect to the virtual straight line vsl. According to this configuration, it is possible to effectively avoid a situation where, when the coupling portion 33 is contracted, an intermediate portion of the coupling portion 33 makes contact with the human body.

A configuration may be adopted in which, unlike in the example shown, when maximally extended, the coupling portion 33 is located on the virtual straight line vsl, or the intermediate connection position mj is locatable on either side with respect to the virtual straight line vsl. In the muscular strength assisting apparatus 10 configured as above, preferably, provided that the coupling portion 33 is maximally extended when the muscular strength assisting apparatus 10 is worn on the human body during actual use, the intermediate connection position mj is located on one side with respect to the virtual straight line vsl, particularly, on the same side as the rotary member 50, which is an opposite side to the human body. In this case, it is possible to effectively avoid a situation where, during use of the muscular strength assisting apparatus 10, the intermediate portion of the coupling portion 33 makes contact with the human body.

Next, a description is given of the driver 40. In the example shown, the driver 40 has the rotary member 50, a retention portion 55 capable of retaining the rotary member 50, and an operation portion 65 that acts on the retention portion 55. The rotary member 50 is rotatably supported to the first attachment tool 20 and connected to the second attachment tool 30. The retention portion 55 retains a rotation position of the rotary member 50. In other words, the retention portion 55 restricts a relative operation between the retention portion 55 and the rotary member 50, particularly, relative rotation therebetween. The operation portion 65 functions as a so-called actuator and acts on the retention portion 55. The operation portion 65 acts on the retention portion 55, and thus a force for stopping (braking) rotation or causing rotation of the rotary member 50 with respect to the first attachment tool 20 is exerted from the retention portion 55 on the rotary member 50.

Furthermore, in the example shown, the retention portion 55 is configured so as to be operable with respect to the first attachment tool 20. The driver 40 further has a biasing member 61 that exerts a force against the force acting from the operation portion 65 on the retention portion 55. A balance between the force supplied from the biasing member 61 and the force supplied from the operation portion 65 is used to control a state of the retention portion 55 and the rotary member 50. The driver 40 further has a control portion 70 that controls an operation of the operation portion 65. The above-described constituent elements constituting the driver 40 will now be described in order.

First, a description is given of the rotary member 50. As shown in Fig. 2, the rotary member 50 has a cylindrical columnar shaft member 51 and a rotary drum 52 increased in diameter from the shaft member 51. The shaft member 51 is rotatably supported by the outer support arm 25 and the inner support arm 26 of the first attachment tool 20. The rotary drum 52 is located between the outer support arm 25 and the inner support arm 26. As shown in Fig. 3, the rotary member 50 is connected to the second attachment tool 30 at an inner end of the shaft member 51. Thus, the connection position of the rotary member 50 to the second attachment tool 30 (the proximal end side connection position pj) is located on the first rotation axis d1 that is the rotation axis of the rotary member 50. The rotary drum 52 is formed in a disc shape. From the operation portion 65 via the retention portion 55, the rotary drum 52 receives a force for stopping rotation with respect to the first attachment tool 20 and a force for rotating with respect to the first attachment tool 20. The rotary drum 52 has a friction layer 52a on a surface layer thereof. The friction layer 52a is a part supposed to make contact with the after-mentioned retention portion 55. The friction layer 52a can be formed of rubber or the like that generates a large friction force between itself and the retention portion 55.

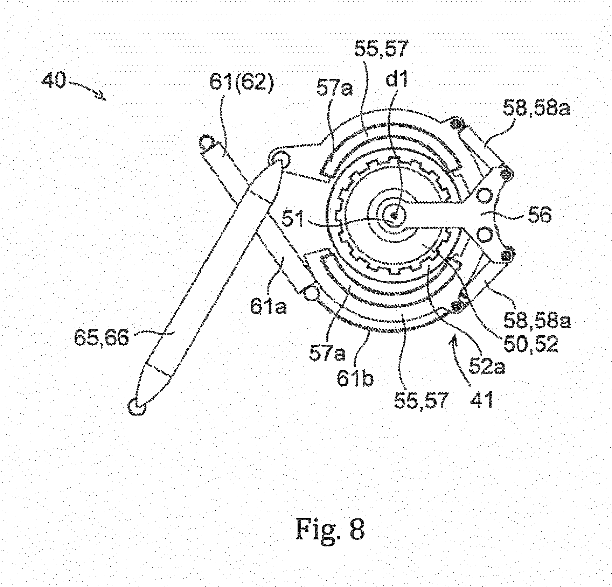

Next, a description is given of the retention portion 55. As shown in Fig. 8 to Fig. 10, the retention portion 55 has a support member 56 operable with respect to the first attachment tool 20 and a swing member 57 supported to the support member 56. In the example shown, the support member 56 is supported to the shaft member 51 of the rotary member 50. The support member 56 is rotatable relative to the shaft member 51 about the first rotation axis d1. Furthermore, a pair of such support members 56 are provided on the shaft member 51. The rotary drum 52 of the rotary member 50 is located between the pair of support members 56 along the first rotation axis d1.

In the example shown, the swing member 57 is supported between respective distal end portions of the pair of support members 56. The swing member 57 is swingable with respect to the support members 56. Particularly in the example shown, a pair of such swing members 57 are provided at distal end portions of the support members 56. The pair of swing members 57 extend from the distal end portions of the support members 56 to different sides from each other. Each of the swing members 57 faces the rotary drum 52 of the rotary member 50 from an outer side in a radial direction about the first rotation axis d1. A major part of an outer peripheral surface of the rotary drum 52 is covered by the pair of swing members 57.

The swing members 57 swing with respect to the support members 56 and thus can come in contact with the rotary drum 52 from the outer side in the radial direction about the first rotation axis d1. The swing members 57 each has a friction body 57a that comes in contact with the rotary member 50 so as to generate a friction force. The friction body 57a makes contact with the friction layer 52a of the rotary drum 52 and thus generates a friction force between itself and the friction layer 52a. The retention portion 55 further has a biasing unit 58 provided between the support members 56 and each of the swing members 57. The biasing unit 58 is applying a bias so that the each of the swing members 57 is separated from the rotary drum 52. In the example shown, the biasing unit 58 is formed of a biasing spring 58a that is a tension spring. Furthermore, the support members 56 are provided with a synchronization gear (not shown) that engages with the pair of swing members 57. The synchronization gear engages with the pair of swing members 57 so that, with respect to the rotary member 50, the pair of swing members 57 operate in synchronization with each other. The synchronization gear makes the pair of swing members 57 operate symmetrically with respect to each other. That is, when one of the swing members 57 is making contact with the rotary drum 52, the other swing member 57 also makes contact with the rotary drum 52. When one of the swing members 57 is separated from the rotary drum 52, the other swing member 57 is also separated from the rotary drum 52.

Next, a description is given of the operation portion 65. As a so-called actuator, the operation portion 65 acts on the retention portion 55. In the example shown, the operation portion 65 has a stretchable member 66 that is stretchable and shrinkable. As shown in Fig. 4, the stretchable member 66 is connected at one end thereof to one of the swing members 57 that is located on an upper side. The stretchable member 66 is connected at the other end thereof to a lower end of the vertical frame 24 of the first attachment tool 20. Depending on a drive force required by the operation portion 65, a plurality of stretchable members 66 can be provided.

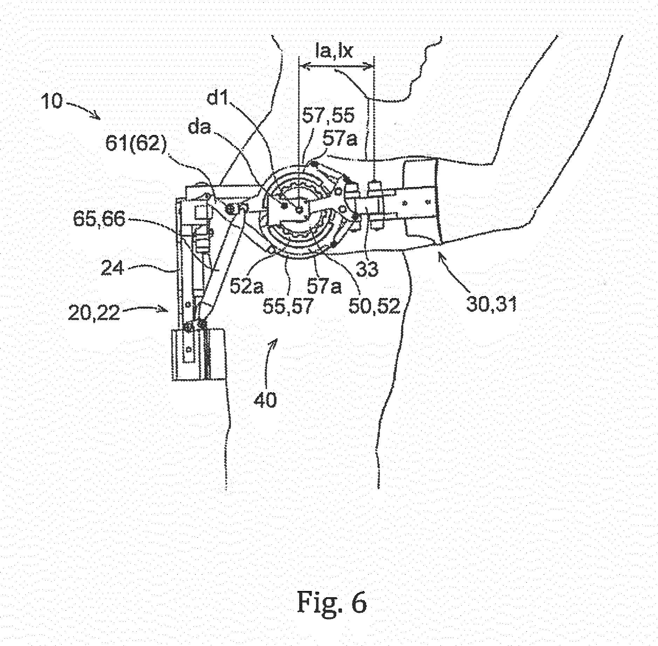

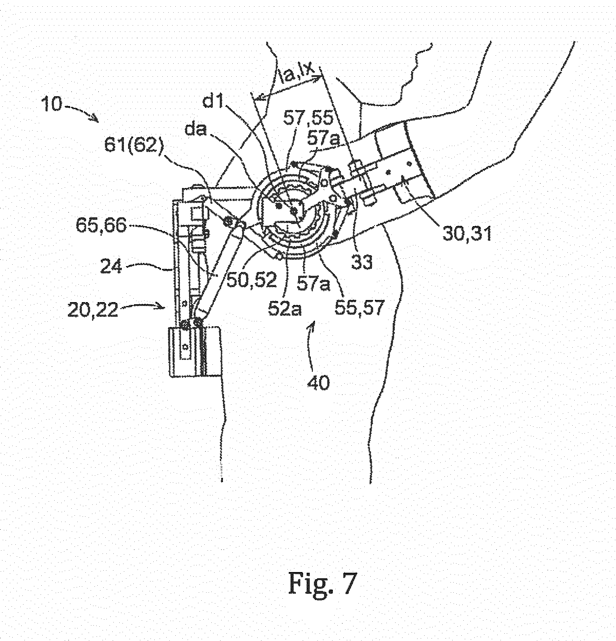

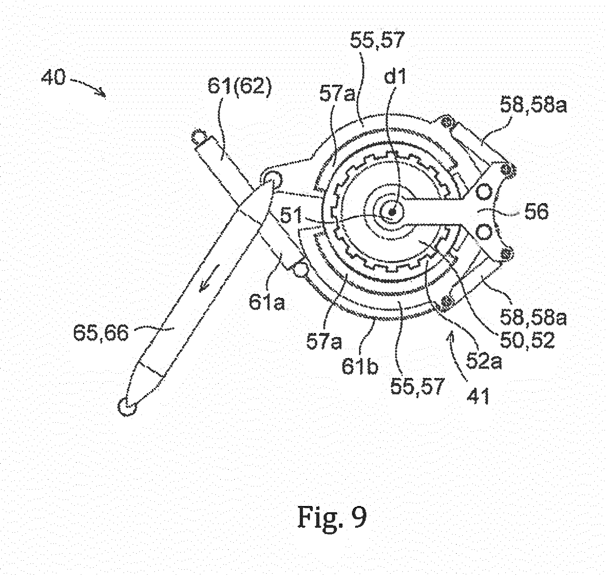

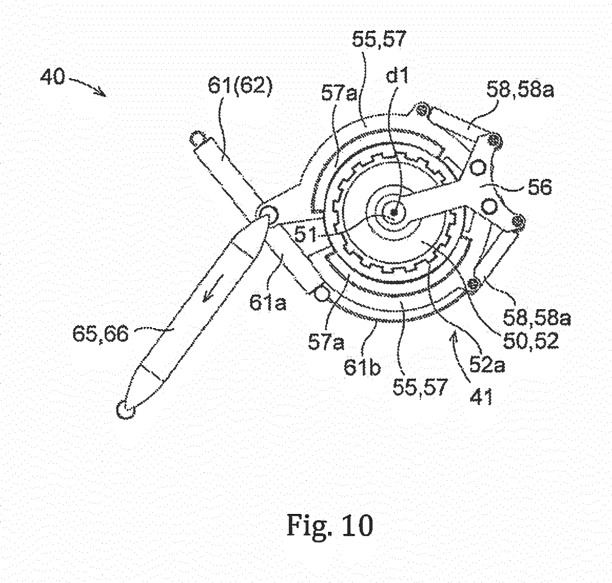

As shown in Fig. 8 and Fig. 9, when the stretchable member 66 shrinks, the operation portion 65 pulls the one of the swing members 57 that is located on the upper side, thus causing the one of the swing members 57 to come in contact with the rotary drum 52 of the rotary member 50. In a state shown in Fig. 6 and Fig. 9, the retention portion 55 retains the rotary member 50, and thus relative rotation between the retention portion 55 and the rotary member 50 is restricted. When the stretchable member 66 shrinks further from this state, the operation portion 65 further pulls the upper one of the swing members 57. As a result, as shown in Fig. 7 and Fig. 10, the operation portion 65 causes the rotary member 50 restricted from relative rotation with respect to the retention portion 55 to rotate together with the retention portion 55 with respect to the first attachment tool 20.

A member expandable and contractible under a fluid pressure can be used as the stretchable member 66. As one specific example, McKibben artificial muscles known as a fluid pressure-type actuator can be used as the stretchable member 66. When formed of McKibben artificial muscles, the stretchable member 66 is supplied inside with a fluid (typically, a gas) and thus becomes increased in diameter and contracted. As a result of this contraction, the stretchable member 66 generates a contraction force. In a case where a member expandable and contractible under a fluid pressure is used as the stretchable member 66, elasticity of the stretchable member 66 itself and compressibility of a fluid itself, which is represented by air, for example, can effectively reduce a restraint feeling of the wearer. In the example shown, the operation portion 65 has a fluid pressure source 67 that can supply a fluid to the stretchable member 66. As shown in Fig. 1, the fluid pressure source 67 configured as, for example, a compressor is supported to the first portion 21 of the first attachment tool 20.

An operation of the operation portion 65 is controlled by the control portion 70. In the example shown in Fig. 1, the control portion 70, together with the fluid pressure source 67, is supported to the first portion 21 of the first attachment tool 20. The control portion 70 controls supply of a fluid (for example, air) from the fluid pressure source 67 to the stretchable member 66 and discharge of the fluid from the stretchable member 66 and thus can control rotation of the rotary member 50 with respect to the first attachment tool 20 and a rotation position thereof.

More specifically, based on the degree of stretching/shrinking of the stretchable member 66 of the operation portion 65, the control portion 70 switches among a free rotation mode shown in Fig. 4, Fig. 5, and Fig. 8, a rotation braking mode shown in Fig. 6 and Fig. 9, and a rotation drive mode shown in Fig. 7 and Fig. 10. In the free rotation mode shown in Fig. 4, Fig. 5, and Fig. 8, the rotary member 50 is freely rotatable with respect to the retention portion 55 and the first attachment tool 20. In the rotation braking mode shown in Fig. 6 and Fig. 9, in a state where relative rotation between the retention portion 55 and the rotary member 50 is restricted, rotation of the rotary member 50 with respect to the first attachment tool 20 is also restricted. In the rotation drive mode shown in Fig. 7 and Fig. 10, in the state where relative rotation between the retention portion 55 and the rotary member 50 is restricted, the rotary member 50 is caused to rotate with respect to the first attachment tool 20.

Next, a description is given of the biasing member 61. The biasing member 61 biases the retention portion 55 in an opposite direction to a direction in which a drive force from the operation portion 65 causes the retention portion 55 to rotate together with the rotary member 50. In a case where it is desired to strengthen a retention force of the retention portion 55 for retaining the rotary member 50, a biasing force of the biasing member 61 may be increased or a plurality of biasing members 61 may be provided.

In the example shown, as shown in Fig. 4, the biasing member 61 is connected at one end thereof to one of the swing members 57 that is located on a lower side. The biasing member 61 is connected at the other end thereof to an upper end of the vertical frame 24 of the first attachment tool 20. In a lateral view of Fig. 4, the biasing member 61 is provided so as to intersect with the stretchable member 66. The biasing member 61 shown has a biasing member body 61a whose end portion is secured to the first attachment tool 20 and a coupling member 61b that couples the biasing member body 61a to the lower one of the swing members 57. A tension spring can be used as the biasing member body 61a, and a metal piece, a wire, or the like can be used as the coupling member 61b.

Next, a description is given of an operation of the muscular strength assisting apparatus 10 having the above-described configuration.

As shown in Fig. 1, first, the first portion 21 of the first attachment tool 20 is attached to the torso by using the attachment belt 11. Furthermore, the attachment portion 31 of the second attachment tool 30 is attached to the upper arm. The muscular strength assisting apparatus 10 is designed so as to reduce a restraint feeling that the wearer might experience. Thus, it is not necessarily required to align rotation axes between the constituent elements of the muscular strength assisting apparatus 10 with the rotation axes of the shoulder joint. This can eliminate the need for precise positioning, thus making it possible for the wearer to wear the muscular strength assisting apparatus 10 with ease and in a short time.

When the wearer wearing the muscular strength assisting apparatus 10 performs a flexion motion or an extension motion about the flexion/extension axis da, the second attachment tool 30 rotates about the first rotation axis d1 with respect to the first attachment tool 20. At this time, the upper arm of the wearer rotates relative to the torso about the flexion/extension axis da. Meanwhile, the attachment portion 31 rotates relative to the first attachment tool 20 about the first rotation axis d1, the attachment portion 31 being attached to the upper arm, and the first attachment tool 20 being mounted to the torso. Here, in a case where the first rotation axis d1 does not agree with the flexion/extension axis da, a movement trajectory of a part of the upper arm about the flexion/extension axis da, the attachment portion 31 being attached to the part, does not agree with a movement trajectory of the attachment portion 31 about the first rotation axis d1. Consequently, the wearer is given a restraint feeling and thus ends up performing flexion or extension of the arm while moving his/her shoulder position.

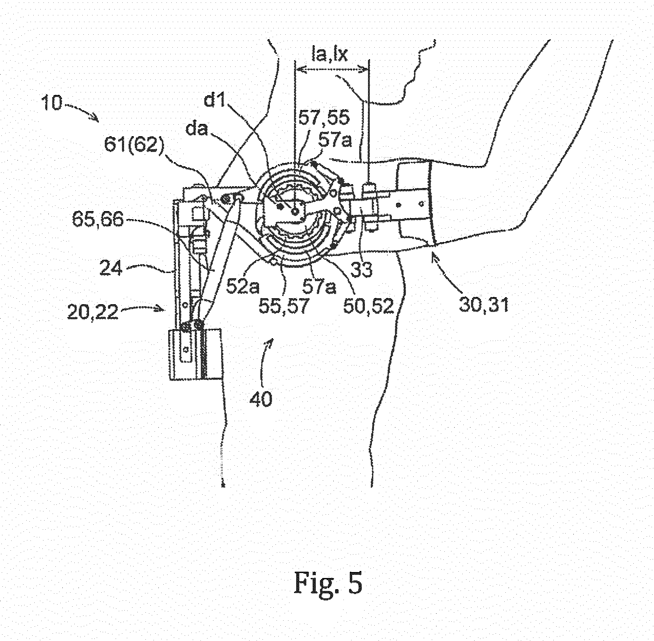

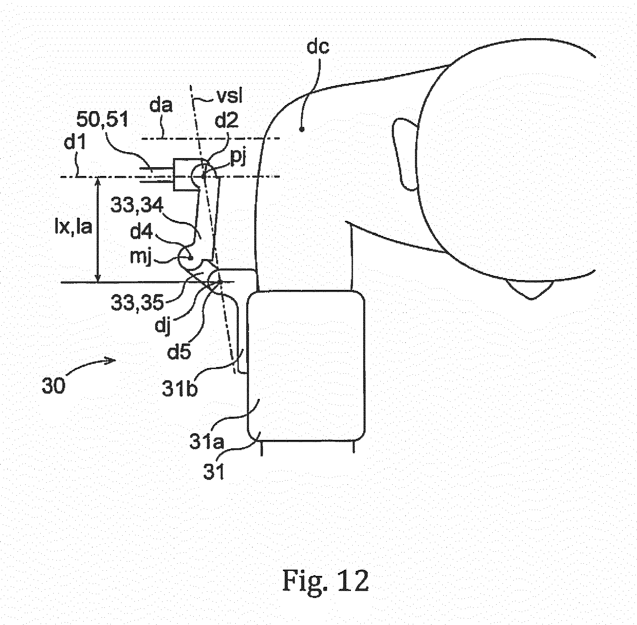

As a solution to this, in the muscular strength assisting apparatus 10 shown, in the plane orthogonal to the first rotation axis d1 that is the rotation axis of the rotary member 50 and including the distal end side connection position dj that is the connection position between the attachment portion 31 and the coupling portion 33 of the second attachment tool 30, that is, in the plane parallel to the plane shown in Fig. 4 and Fig. 5, the distance la between the proximal end side connection position pj that is the connection position of the coupling portion 33 to the rotary member 50 and the distal end side connection position dj that is the connection position of the coupling portion 33 to the attachment portion 31 varies, and the distance lx between the first rotation axis d1 and the distal end side connection position dj varies. In other words, in the plane including the first rotation axis d1 and the distal end side connection position dj, which is shown in Fig. 11 and Fig. 12, the separation spacing la between the proximal end side connection position pj and the distal end side connection position dj along the direction orthogonal to the first rotation axis d1 varies, and the separation spacing lx between the first rotation axis d1 and the distal end side connection position dj along the direction orthogonal to the first rotation axis d1 varies. In the example shown, in a state shown in Fig. 11 and Fig. 4, the separation spacings (distances) la and lx are longer than in a state shown in Fig. 12 and Fig. 5. With the distance la between the proximal end side connection position pj and the distal end side connection position dj varying, in a flexion/extension motion, a movement trajectory of the attachment portion 31 can be made to agree with a moving trajectory of the part of the upper arm, the attachment portion 31 being attached to the part. Thus, it is possible to effectively reduce a restraint feeling of the wearer wearing of the muscular strength assisting apparatus 10 during a flexion/extension motion.

Furthermore, in this embodiment, the rotary member 50, the coupling portion 33, and the attachment portion 31 constitute a mechanism having three degrees of freedom. According to the second attachment tool 30 configured as above, it becomes also possible to adjust the distal end side connection position dj that is the connection position between the attachment portion 31 and the coupling portion 33 in a plane including the first rotation axis d1 that is the rotation axis of the rotary member 50, thus adjusting a position of the attachment portion 31 in two directions, namely, a direction parallel to the first rotation axis d1 and a direction orthogonal to the first rotation axis d1 while adjusting an orientation of the attachment portion 31. In this case, not only in a case where the first rotation axis d1 has moved parallel to the flexion/extension axis da but also in a case where the first rotation axis d1 is inclined with respect to the flexion/extension axis da. it is possible to achieve a flexion motion and an extension motion while reducing a restraint feeling.

Furthermore, human body joints enable rotation motions about a plurality of axes orthogonal to each other. For example, a single shoulder joint enables a flexion/extension motion and an adduction/abduction motion as relative rotation motions about the axes da and db orthogonal to each other. In order to prevent such various types of motions from being restrained, in a muscular strength assisting apparatus disclosed in Japanese Patent Application Publication No. 2009-268839, a plurality of relative rotation axes are provided, and the plurality of relative rotation axes are set to intersect with each other at one place. Further, a wearer wears the muscular strength assisting apparatus so that a position at which the plurality of relative rotation axes intersect with each other is located on a joint of the wearer. In the muscular strength assisting apparatus, however, setting the plurality of rotation axes to intersect with each other at one place leads to a complicated configuration and a size increase of the muscular strength assisting apparatus. Moreover, it is difficult to keep successfully arranging the position at which the plurality of rotation axes of the muscular strength assisting apparatus intersect with each other on the joint of the wearer.

On the other hand, according to the muscular strength assisting apparatus 10 shown, also in an adduction/abduction motion and an internal/external rotation motion, it is possible to effectively reduce a restraint feeling that a wearer might experience.

First, when the wearer wearing the muscular strength assisting apparatus 10 performs an adduction motion or an abduction motion about the adduction/abduction axis db, as shown in Fig. 13, the second attachment tool 30 rotates with respect to the first attachment tool 20 about the second rotation axis d2. At this time, the upper arm of the wearer rotates relative to the torso about the adduction/abduction axis db. Meanwhile, the attachment portion 31 attached to the upper arm rotates relative to the first attachment tool 20 mounted to the torso about the second rotation axis d2. Here, in a case where the second rotation axis d2 does not agree with the adduction/abduction axis db, a movement trajectory of the part of the upper arm about the adduction/abduction axis db, the attachment portion 31 being attached to the part, does not agree with a movement trajectory of the attachment portion 31 about the second rotation axis d2. Consequently, the wearer is given a restraint feeling and thus ends up performing adduction or abduction of the arm while moving his/her shoulder position.

However, in the muscular strength assisting apparatus 10 shown, in the plane orthogonal to the second rotation axis d2 and including the distal end side connection position dj, that is, in the plane shown in Fig. 11 and Fig. 13, the distance lb between the proximal end side connection position pj and the distal end side connection position dj varies, and a distance ly between the second rotation axis d2 and the distal end side connection position dj varies. Furthermore, in other words, in a plane including the second rotation axis d2 and the distal end side connection position dj, the separation spacing lb between the proximal end side connection position pj and the distal end side connection position dj along a direction orthogonal to the second rotation axis d2 varies, and the separation spacing ly between the second rotation axis d2 and the distal end side connection position dj along the direction orthogonal to the second rotation axis d2 varies. In the example shown, in the state shown in Fig. 11, the separation spacings (distances) lb and ly are longer than in a state shown in Fig. 13. With the distance lb between the proximal end side connection position pj and the distal end side connection position dj varying, in an adduction/abduction motion, a movement trajectory of the attachment portion 31 can be made to agree with a movement trajectory of the part of the upper arm, the attachment portion 31 being attached to the part. Thus, it is possible to effectively reduce a restraint feeling of the wearer wearing the muscular strength assisting apparatus 10 during an adduction/abduction motion.

In the example shown, the proximal end side connection position pj that is the connection position of the coupling portion 33 to the rotary member 50 is located on the first rotation axis d1 that is the rotation axis of the rotary member 50. Therefore, in the plane orthogonal to the second rotation axis d2 and including the distal end side connection position dj, the distance lb between the proximal end side connection position pj and the distal end side connection position dj agrees with the distance ly between the second rotation axis d2 and the distal end side connection position dj.

Next, a description is given of an operation of the driver 40.

As mentioned above, the muscular strength assisting apparatus 10 shown can be maintained in any of the free rotation mode shown in Fig. 4, Fig. 5, and Fig. 8, the rotation braking mode shown in Fig. 6 and Fig. 9, and the rotation drive mode shown in Fig. 7 and Fig. 10.

First, in the free rotation mode shown in Fig. 4, Fig. 5, and Fig. 8, a fluid in the stretchable member 66 is discharged, and thus the stretchable member 66 is in a stretched state. Thus, the swing members 57 of the retention portion 55 are not being subjected to a force from the stretchable member 66 toward the rotary drum 52. On the other hand, a biasing force (a tension force) from the biasing member 61 causes the retention portion 55 to rotate in a counterclockwise direction in Fig. 8. The coupling member 61b of the biasing member 61, however, is connected to the vicinity of a swing center of the one of the swing members 57 that is located on the lower side. Thus, in a state shown in Fig. 8, the swing members 57 are not being subjected to a force for bringing the swing members 57 in contact with the rotary member 50 from the biasing member 61, either. By the biasing unit 58, each of the swing members 57 is biased in a direction away from the rotary member 50. Thus, in the free rotation mode, rotation of the rotary member 50 about the first rotation axis d1 is not restricted by the retention portion 55, and the rotary member 50 can freely rotate about the first rotation axis d1.

In the free rotation mode, the second attachment tool 30 is freely rotatable with respect to the first attachment tool 20. Thus, the wearer of the muscular strength assisting apparatus 10 can freely rotate the upper arm with respect to the torso about the first rotation axis d1, the attachment portion 31 of the second attachment tool 30 being mounted to the upper arm. That is, the wearer can perform a flexion motion and an extension motion without being restrained by the muscular strength assisting apparatus 10.

Next, in the rotation breaking mode shown in Fig. 6 and Fig. 9, a fluid is supplied from the fluid pressure source 67 into the stretchable member 66, and thus the stretchable member 66 becomes contracted. When the stretchable member 66 becomes contracted, the operation portion 65 acts on the one of the swing members 57 that is disposed on the upper side so as to pull the one of the swing members 57. As shown in Fig. 9, a force exerted from the stretchable member 66 on the swing members 57 causes the swing members 57 to operate toward the rotary drum 52 of the rotary member 50. As shown in Fig. 9, the friction body 57a of each of the swing members 57 comes in contact with the friction layer 52a forming an outer peripheral surface of the rotary drum 52. The retention portion 55 retains the rotary member 50 by using a friction force between the friction body 57a and the friction layer 52a. As a result, relative rotation between the retention portion 55 and the rotary member 50 is restricted, and thus a rotation position of the rotary member 50 is maintained.

In the rotation braking mode, relative rotation of the second attachment tool 30 with respect to the first attachment tool 20 is restricted. That is, the muscular strength assisting apparatus 10 assists in maintaining the upper arm lifted as shown in Fig. 6 at that lifted position and thus can effectively suppress coming down of the upper arm. Though connected to the biasing member 61 and the operation portion 65, the retention portion 55 is still rotatable with respect to the first attachment tool 20. Thus, the wearer can further lift the upper arm from the state shown in Fig. 6 against a biasing force of the biasing member 61.

In the rotation drive mode shown in Fig. 7 and Fig. 10, a further amount of fluid is supplied from the fluid pressure source 67 into the stretchable member 66, and thus the stretchable member 66 becomes further contracted. When the stretchable member 66 becomes further contracted, the operation portion 65 further pulls the one of the swing members 57 that is disposed on the upper side. In a state where the swing members 57 have been brought in contact with the rotary member 50, a force exerted from the stretchable member 66 on the swing members 57 causes the retention potion 55 to further rotate together with the rotary member 50 about the first rotation axis d1 against a biasing force of the biasing member 61. In Fig. 7 and Fig. 10, in a state where relative rotation between the retention portion 55 and the rotary member 50 is restricted, the retention portion 55 and the rotary member 50 rotate counterclockwise in synchronization with each other.

In the rotation drive mode, the second attachment tool 30 is driven to rotate relative to the first attachment tool 20. That is, the muscular strength assisting apparatus 10 assists in lifting the upper arm, for example, from the state shown in Fig. 6 and Fig. 9 to a state shown in Fig. 7 and Fig. 10.

To end the rotation drive mode, the operation portion 65 stops a drive force for causing the rotary member 50 to rotate with respect to the first attachment tool 20. At this time, the operation portion 65 may maintain supply of a drive force for restricting relative rotation between the retention portion 55 and the rotary member 50. That is, after ending the rotation drive mode, a transition may be made to the rotation braking mode instead of the free rotation mode.

In this embodiment described so far, the second attachment tool 30 has the attachment portion 31 attached to the human body and the coupling portion 33 that couples the rotary member 50 to the attachment portion 31 so that, as the rotary member 50 rotates with respect to the first attachment tool 20, the attachment portion 31 rotates with respect to the first attachment tool 20. In the plane orthogonal to the first rotation axis d1 of the rotary member 50 and including the connection position dj between the attachment portion 31 and the coupling portion 33, the distance la between the connection position pj of the coupling portion 33 to the rotary member 50 and the connection position dj of the coupling portion 33 to the attachment portion 31 varies. In other words, in the plane including the rotation axis d1 of the rotary member 50, a distance between the rotation axis d1 and the connection position dj varies. That is, in the plane including the rotation axis d1 of the rotary member 50, a position of the attachment portion 31 can be made to vary. Thus, even when the rotation axis d1 of the rotary member 50 is misaligned from the rotation axis da of an action using the joint, which is sought to be assisted by exerting a force on the rotary member 50, it is possible to achieve the action using the joint while reducing a restraint feeling. Furthermore, since it is not required that the rotation axis d1 of the rotary member 50 precisely agree with the rotation axis da of the joint, it becomes possible to obtain a simplified and smaller-sized configuration of the muscular strength assisting apparatus 10, and positioning of the muscular strength assisting apparatus 10 on the human body can be facilitated. The configuration for reducing a restraint feeling is useful not only for an active-type muscular strength assisting apparatus (a muscular strength assisting apparatus that actively assists actions) but also for a passive-type muscular strength assisting apparatus (a muscular strength assisting apparatus that assists in maintaining states (postures)).

Furthermore, in this embodiment, the rotary member 50, the coupling portion 33, and the attachment portion 31 constitute the mechanism having three degrees of freedom. According to the coupling portion 33 configured as above, it can be made possible to adjust the connection position dj between the attachment portion 31 and the coupling portion 33 in the plane including the rotation axis d1 of the rotary member 50, thus adjusting a position of the attachment portion 31 in two directions, namely, the direction parallel to the rotation axis d1 of the rotary member 50 and the direction orthogonal to the rotation axis d1 of the rotary member 50 while adjusting an orientation of the attachment portion 31. In this case, even when the rotation axis d1 of the rotary member 50 is misaligned from the rotation axis da of an action using the joint, which is sought to be assisted by exerting a force on the rotary member 50, it is possible to achieve the action using the joint while reducing a restraint feeling. Furthermore, in this case, also for actions other than such an action using the joint, which is sought to be assisted by exerting a force on the rotary member 50 (in the above-mentioned example, an adduction/abduction motion other than a flexion/extension motion), a restraint feeling can be reduced.

In this embodiment, the coupling portion 33 has the first member 34 operably connected to the rotary member 50 and the second member 35 operably connected to the first member 34 and operably connected to the attachment portion 31. According to the coupling portion 33 configured as above, it is possible to achieve a link mechanism with three degrees of freedom by using a simple and less costly configuration.

In this embodiment, a connection is made in any one of the following combinations: the rotary member 50 and the first member 34 of the coupling portion 33; the first member 34 of the coupling portion 33 and the second member 35 of the coupling portion 33; and the second member 35 of the coupling portion 33 and the attachment portion 31, so as to enable rotation about the axis d2 non-parallel to the rotation axis d1 of the rotary member 50. According to the coupling portion 33 configured as above, also for actions other than an action using the joint, which is sought to be assisted by exerting a force on the rotary member 50, a restraint feeling can be reduced. That is, the coupling portion 33 functions not only as a unit for reducing a restraint feeling with respect to such an action using the joint, which is sought to be assisted by exerting a force on the rotary member 50, but also as a unit for enabling actions other than the action using the joint. In addition, also for the other actions, a restraint feeling can be reduced.

In this embodiment, relative rotation between the first attachment tool 20 and the second attachment tool 30 about the rotation axis d1 of the rotary member 50 enables a flexion motion or an extension motion using the joint, and relative rotation of the one of the above-described combinations about the axis d2 non-parallel to the rotation axis d1 of the rotary member 50 enables an adduction motion or an abduction motion using the joint. Thus, it is possible to achieve a flexion motion and an extension motion using the joint while assisting these motions without giving an excessive degree of restraint feeling, and at the same time, to achieve an adduction motion and an abduction motion using that joint while reducing a restraint feeling.

In this embodiment, the first attachment tool 20 has the first portion 21 and the second portion 22 connected so as to be rotatable with respect to the first portion 21. The rotation axis d3 between the first portion 21 and the second portion 22 is non-parallel to the rotation axis d1 of the rotary member 50 and also non-parallel to the rotation axis d2 of the one of the above-described combinations. According to the muscular strength assisting apparatus 10, it is possible to achieve actions about the three different axes da, db, and dc related to one joint. This makes it possible to further effectively reduce a restraint feeling.

In this embodiment, in the plane including the rotation axis d1 of the rotary member 50 and the connection position dj between the attachment portion 31 and the coupling portion 33, the connection position mj between the first member 34 and the second member 35 of the coupling portion 33 is always located on one side with respect to the straight line vsl passing through the connection position pj of the coupling portion 33 to the rotary member 50 and the connection position dj of the coupling portion 33 to the attachment portion 31. Thus, when the muscular strength assisting apparatus 10 is worn on the human body, even in a state where, in the plane including the rotation axis d1 of the rotary member 50 and the connection position dj between the attachment portion 31 and the coupling portion 33, a distance between the connection position pj of the coupling portion 33 to the rotary member 50 and the connection position dj of the coupling portion 33 to the attachment portion 31 is maximally long, the connection position mj between the first member 34 and the second member 35 of the coupling portion 33 is located on one side with respect to the straight line vsj passing through the connection position pj of the coupling portion 33 to the rotary member 50 and the connection position dj of the coupling portion 33 to the attachment portion 31. That is, the connection position mj between the first member 34 and the second member 35 of the coupling portion 33 can be made to always protrude to a side away from the human body. As a result, the coupling portion 33 can be effectively prevented from making contact with the human body when an action using the joint is performed.

In this embodiment, the connection position pj of the coupling portion 33 to the rotary member 50 is located on the rotation axis d1 of the rotary member 50. It is, therefore, possible to effectively suppress a situation where a difference between a distance from the joint to the attachment portion 31 and a distance from the connection position pj of the coupling portion 33 to the rotary member 50 to the attachment portion 31 varies with rotation. Thus, a smooth action using the joint can be enabled with a restraint feeling further eliminated.

In this embodiment, there is provided the operation portion 65 that, in a state where relative rotation between the retention portion 55 and the rotary member 50 is restricted, acts on the retention portion 55 so that the rotary member 50 rotates together with the retention portion 55 with respect to the first attachment tool 20. Thus, it is possible not only to simply retain a relative position between the first attachment tool 20 and the second attachment tool 30 but also to cause the operation portion 65 to act on the retention portion 55 so that the second attachment portion 30 operates with respect to the first attachment tool 20. That is, actions of the wearer can be actively assisted, and compared with an apparatus that simply retains positions, muscular strength assisting performance can be remarkably improved. Moreover, there is provided the control portion 70 that controls an operation of the operation portion 65. The control portion 70 switches among the free rotation mode in which the rotary member 50 is rotatable with respect to the retention portion 55, the rotation braking mode in which, in a state where relative rotation between the retention portion 55 and the rotary member is restricted, rotation of the rotary member 50 with respect to the first attachment tool 20 is restricted, and the rotation drive mode in which, in the state where the relative rotation between the retention portion 55 and the rotary member 50 is restricted, the rotary member 50 is caused to rotate with respect to the first attachment tool. In the rotation drive mode, it is possible to fulfill excellent muscular strength assisting performance. Furthermore, in the free rotation mode, it is possible to significantly reduce a restraint feeling that might be given to the wearer and a strain on the wearer.

In this embodiment, the operation portion 65 supplies a drive force to the retention portion 55 so as to restrict relative rotation between the retention portion 55 and the rotary member 50. That is, the operation portion 65 supplies the retention portion 55 with a drive force for making the retention portion 55 retain the rotary member 50 and a drive force for causing the retention portion 55 retaining the rotary member 50 to rotate together with the rotary member 50. By use of the single operation portion 65, both retention of a position of the second attachment tool 30 with respect to the first attachment tool 20 and an operation of the second attachment tool 30 with respect to the first attachment tool 20 can be achieved, and thus the muscular strength assisting apparatus 10 can be reduced in size and weight.

In this embodiment, in the rotation drive mode, the control portion 70 controls the operation portion 65 to supply the retention portion 55 with a drive force for restricting relative rotation between the retention portion 55 and the rotary member 50 and a drive force for causing the rotary member 50 to rotate with respect to the first attachment tool 20. That is, the single operation portion 65 outputs both the drive force for restricting relative rotation between the retention portion 55 and the rotary member 50 and the drive force for causing the rotary member to rotate with respect to the first attachment tool. Thus, the muscular strength assisting apparatus can be reduced in size and weight.

In this embodiment, when ending the rotation drive mode, the control portion 70 controls the operation portion 65 to make a transition to the rotation braking mode by stopping the drive force for causing the rotary member 50 to rotate with respect to the first attachment tool 20, while maintaining supply of the drive force for restricting relative rotation between the retention portion 55 and the rotary member 50. According to this embodiment having the above-described configuration, upon completing assistance with an action of the wearer, the muscular strength assisting apparatus 10 assists in retaining the human body of the wearer in its immediately previous state. Thus, it is possible to avoid an abrupt increase in strain on the wearer.

In this embodiment, the retention portion 55 has the friction body 57a that comes in contact with the rotary member 50 so as to generate a friction force. That is, a friction force is used to restrict relative rotation between the rotary member 50 and the retention portion 55.

According to this embodiment, there is provided the biasing member 61 that biases the retention portion 55 in an opposite direction to a direction in which a drive force from the operation portion 65 causes the retention portion 55 to rotate together with the rotary member 50. Thus, depending on a magnitude of a force acting on the retention portion 55 from the operation portion 65, a position at which the retention portion 55, together with the rotary member 50, stops rotation with respect to the first attachment tool 20 can be controlled. That is, a position at which the second attachment tool 30 is secured with respect to the first attachment tool 20 can be made to vary as appropriate.

According to this embodiment, the retention portion 55 comes in contact with the rotary member 50 from a radial direction about the rotation axis d1 of the rotary member 50 so as to restrict relative rotation between the retention portion 55 and the rotary member 50. That is, the retention portion 55 and the rotary member 50 constitute a drum brake mechanism 41 and thus can be configured simply at a reduced cost.

According to this embodiment, the retention portion 55 has the support member 56 rotatable relative to the rotary member 50 about the rotation axis d1 of the rotary member 50 and the swing member 57 swingably retained to the support member 56. The operation portion 65 is connected to the swing member 57 and applies, to the swing member 57, a force for pulling the swing member 57 so that the swing member 57 comes in contact with the rotary member 50 from the radial direction about the rotation axis d1 of the rotary member 50. Furthermore, the operation portion 65 increases the force to be applied to the swing member 57 from a state where the swing member 57 is in contact with the rotary member 50 so that the rotary member 50 restricted from relative rotation with respect to the swing member 57 is caused to rotate together with the retention portion 55 with respect to the first attachment tool 20. This simplifies an operation and control of the operation portion 65, and thus the operation portion 65 and the control portion 70 can be configured simply at a reduced cost.

While the foregoing description has been made based on one embodiment illustrating the present invention, the present invention is not limited thereto and can be implemented in various other aspects. The following describes one modification example. In the following description, parts that can be configured similarly to those in the foregoing embodiment are denoted by the same reference characters as those used for corresponding parts in the foregoing embodiment, and duplicate descriptions thereof are omitted.

The foregoing embodiment has described a specific configuration of the retention portion 55 merely as an example. While the foregoing embodiment has shown an example in which the retention portion 55, together with the rotary drum 52, constitutes the drum brake mechanism 41, and the swing member 57 of the retention portion 55 comes in contact with the rotary drum 52 from the outer side in the radial direction, there is no limitation thereto. A configuration may also be adopted in which the rotary drum 52 is formed in a barrel shape, and the retention portion disposed inside the rotary drum 52 comes in contact with the rotary drum 52 from an inner side in the radial direction so as to restrict relative rotation between the retention portion 55 and the rotary member 50. Furthermore, a configuration may also be adopted in which the retention portion is formed as a ratchet mechanism, and in a state where relative rotation with respect to the rotary member 50 is restricted, a drive force from the operation portion 65 causes the retention portion to rotate together with the rotary member 50.

Furthermore, while the foregoing embodiment has shown an example in which the retention portion 55 is rotatably supported on the shaft member 51 of the rotary member 50, there is no limitation thereto. A configuration may also be adopted in which the support member 56 of the retention portion 55 is secured with respect to the shaft member 51. Furthermore, the support member 56 of the retention portion 55 may be supported to the first attachment tool 20 or may be supported to the second attachment tool 30.

The foregoing embodiment has described a specific configuration of the operation portion 65 merely as an example. While the foregoing embodiment has shown an example in which the operation portion 65 applies, to the swing member 57, a force for pulling the swing member 57 so that the swing member 57 comes in contact with the rotary member 50, there is no limitation thereto. A configuration may also be adopted in which the operation portion 65 applies, to the swing member 57, a force for pushing the swing member 57 so that the swing member 57 comes in contact with the rotary member 50. In the first place, there is no limitation to the operation portion 65 having the stretchable member 66, and various types of actuators can be used as the operation portion 65.

Furthermore, while the foregoing embodiment has shown an example in which, in the rotation drive mode, the muscular strength assisting apparatus 10 assists only an action of lifting an arm upward and does not assist an action of lowering the arm, there is no limitation thereto, and the muscular strength assisting apparatus 10 may be configured to assist an action of lowering an arm in the rotation drive mode. For example, in the foregoing embodiment, a force acting on the swing member 57 from the operation portion 65 is set to be weaker than a biasing force of the biasing member 61 and yet sufficient to be able to restrict relative rotation between the retention portion 55 and the rotary member 50, and thus an action of lowering an arm can be assisted.

Moreover, while the foregoing embodiment has shown an example in which the biasing member 61 is provided between the retention portion 55 and the first attachment tool 20, there is no limitation thereto, and an auxiliary actuator 62 can be used in place of the biasing member 61. According to this modification example, depending on a magnitude of a force acting on the retention portion 55 from the operation portion 65 and a magnitude of a force acting on the retention portion 55 from the auxiliary actuator 62, a position at which the retention portion 55, together with the rotary member 50, stops rotation with respect to the first attachment tool 20 can be controlled with a higher degree of freedom. That is, a position at which the second attachment tool 30 is secured with respect to the first attachment tool 20 can be adjusted with a higher degree of freedom. Furthermore, it becomes also possible to assist in lowering an upper arm.

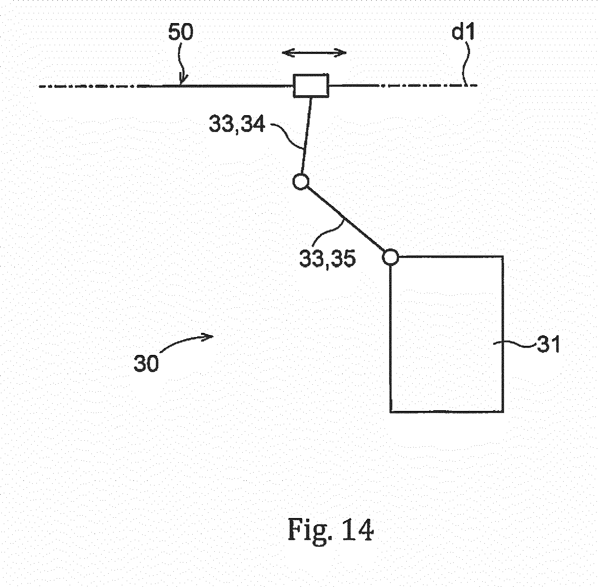

Moreover, the foregoing embodiment has described a specific configuration of the coupling portion 33 of the second attachment tool 30 merely as an example, and the coupling portion 33 can be modified, for example, as shown in Fig. 14 to Fig. 17. In any of modification examples shown in Fig. 14 to Fig. 17, the attachment portion 31 can perform relative operations with three degrees of freedom with respect to the rotary member 50.

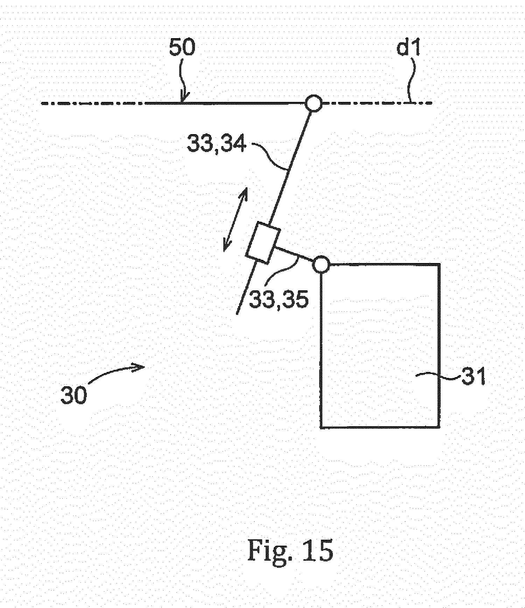

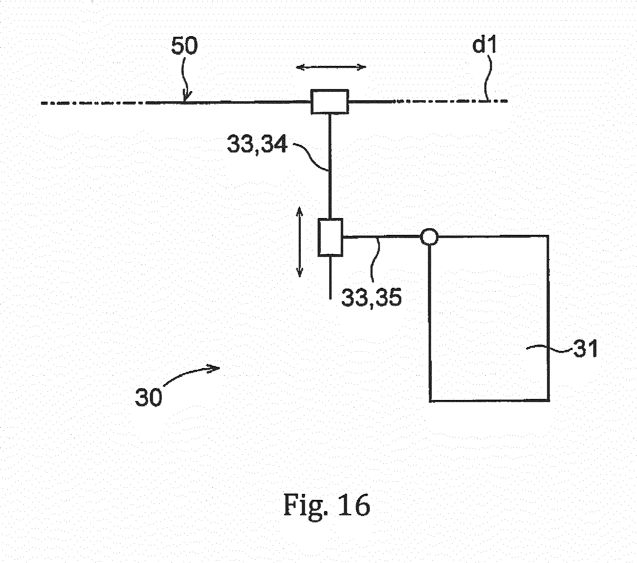

The example shown in Fig. 14 is different from the foregoing embodiment in that the first member 34 of the coupling portion 33 is connected to the rotary member 50 so as to be slidable in a direction parallel to the first rotation axis d1. The example shown in Fig. 15 is different from the foregoing embodiment in that the second member 35 of the coupling portion 33 is connected to the first member 34 so as to be slidable in a longitudinal direction of the first member 34. The example shown in Fig. 16 is different from the foregoing embodiment in that the first member 34 of the coupling portion 33 is connected to the rotary member 50 so as to be slidable in the direction parallel to the first rotation axis d1 and in that the second member 35 of the coupling portion 33 is connected to the first member 34 so as to be slidable in the longitudinal direction of the first member 34.

In the example shown in Fig. 18, the coupling portion 33 includes a fluid pressure cylinder 37. The coupling portion 33 including the fluid pressure cylinder 37 is rotatably connected at one end thereof to the rotary member 50 and rotatably connected at the other end thereof to the attachment portion 31. According to the modification example shown in Fig. 18, also for actions (for example, an adduction/abduction motion) other than an action (for example, a flexion/extension motion) using a joint, which is sought to be assisted by exerting a force on the rotary member 50, muscle strength assistance can be achieved.

While several modification examples with respect to the foregoing embodiment have thus been described, needless to say, plural ones of these modification examples can be combined as appropriate, and such combinations are also applicable to the present invention.

## Claims

1. A muscular strength assisting apparatus, comprising:
a first attachment tool mounted to one of two parts of a human body, which are connected together via a joint;
a second attachment tool mounted to the other part of the human body; and
a rotary member rotatably supported to the first attachment tool and connected to the second attachment tool, the rotary member receiving a force for stopping or rotating with respect to the first attachment tool,
wherein the second attachment tool includes:
an attachment portion attached to the human body; and
a coupling portion coupling the rotary member to the attachment portion so that the attachment portion rotates with respect to the first attachment tool as the rotary member rotates with respect to the first attachment tool, and
wherein in a plane orthogonal to a rotation axis of the rotary member and including a connection position between the attachment portion and the coupling portion, a distance between the rotation axis of the rotary member and the connection position of the coupling portion to the attachment portion varies.

2. The muscular strength assisting apparatus according to claim 1, wherein the rotary member, the coupling portion, and the attachment portion constitute a mechanism having three degrees of freedom.

3. The muscular strength assisting apparatus according to claim 1 or 2, wherein the coupling portion includes:
a first member operably connected to the rotary member; and
a second member operably connected to the first member and operably connected to the attachment portion.

4. The muscular strength assisting apparatus according to claim 3, wherein a connection is made in any one of following combinations: the rotary member and the first member of the coupling portion; the first member of the coupling portion and the second member of the coupling portion; and the second member of the coupling portion and the attachment portion, so as to enable rotation about an axis non-parallel to the rotation axis of the rotary member.

5. The muscular strength assisting apparatus according to claim 4, wherein relative rotation between the first attachment tool and the second attachment tool about the rotation axis of the rotary member enables a flexion motion or an extension motion using the joint, and
relative rotation of the one of the combinations about the axis non-parallel to the rotation axis of the rotary member enables an adduction motion or an abduction motion using the joint.

6. The muscular strength assisting apparatus according to claim 4 or 5, wherein the first attachment tool includes:
a first portion; and
a second portion operably connected to the first portion, and
a rotation axis between the first portion and the second portion is non-parallel to the rotation axis of the rotary member and also non-parallel to the rotation axis of the one of the combinations.

7. The muscular strength assisting apparatus according to any one of claims 3 to 6, wherein the rotary member and the first member of the coupling portion are rotatably connected to each other,
the first member of the coupling portion and the second member of the coupling portion are rotatably connected to each other, and
the second member of the coupling portion and the attachment portion are rotatably connected to each other.

8. The muscular strength assisting apparatus according to claim 7, wherein, in a plane including the rotation axis of the rotary member and the connection position between the attachment portion and the coupling portion, a connection position between the first member and the second member of the coupling portion is located on one side with respect to a straight line passing through a connection position of the coupling portion to the rotary member and the connection position of the coupling portion to the attachment portion.

9. The muscular strength assisting apparatus according to any one of claims 1 to 8, wherein the coupling portion includes a fluid pressure cylinder.

10. The muscular strength assisting apparatus according to any one of claims 1 to 9, wherein the connection position of the coupling portion to the rotary member is located on the rotation axis of the rotary member.
